# EUROPÄISCHE PATENTSCHRIFT

(11) **EP 0 739 604 B1**
(45) Veröffentlichungstag und Bekanntmachung des Hinweises auf die Patenterteilung: **26.05.1999**
(21) Anmeldenummer: 95109492.9
(22) Anmeldetag: 20.06.1995
(51) Int. Cl.: A61B 17/00

(54) **Vorrichtung zum Entfernen von Gewebe oder dergleichen aus der Bauchhöhle**
Device for removing tissues out of the abdominal cavity
Dispositif pour retirer des tissus hors de la cavité abdominale

(30) Priorität: 26.04.1995 DE 19515280
(43) Veröffentlichungstag der Anmeldung: 30.10.1996
(73) Patentinhaber: Riek, Siegfried, Dr. med., D-78667 Villingendorf (DE); Bachmann, Karl-Heinz, D-78667 Villingendorf (DE); Gaiselmann, Thomas, D-78667 Villingendorf (DE)
(72) Erfinder: Riek, Siegfried, Dr. med., D-78667 Villingendorf (DE); Bachmann, Karl-Heinz, D-78667 Villingendorf (DE); Gaiselmann, Thomas, D-78667 Villingendorf (DE)
(74) Vertreter: Patentanwälte Westphal, Mussgnug & Partner

(56) Entgegenhaltungen:
- EP-A- 0 542 103
- DE-U- 9 205 604
- US-A- 5 037 379

## Beschreibung

Die Erfindung betrifft eine Vorrichtung zum Entfernen von Gewebe oder dergleichen aus der Bauchhöhle gemäß dem Oberbegriff des Anspruchs 1.

In der minimal invasiven Chirurgie erfolgt der operative Zugang zur Bauchhöhle durch in die Bauchdecke eingesetzte Trokarhülsen. Der Vorteil der minimal invasiven Chirurgie besteht in der kleinen Operationswunde und den daraus resultierenden geringeren Operationsschmerzen, kürzeren Rekonvaleszenzzeiten und unauffälligeren Operationnarben. Die fortschreitende Operationstechnik der minimal invasiven Chirurgie ermöglicht auch das operative Entfernen von größeren Gewebsteilen, z.B. von Zysten, Tumoren oder sogar Organen oder Organteilen, wie der Gebärmutter, der Niere oder von Darmteilen. Werden solche Gewebsteile, Organe usw. -in der folgenden Anmeldung zur Vereinfachung zusammenfassend als "Gewebe" bezeichnet- in der Bauchhöhle operativ entfernt, so bleibt das Problem, daß dieses Gewebe häufig einen größeren Durchmesser aufweist als die Trokarhülse und somit nicht durch diese Trokarhülse aus der Bauchhöhle entfernt werden kann.

Eine Möglichkeit zur Beseitigung dieses Problems besteht darin, das Gewebe in der Bauchhöhle zu zerkleinern. Dieses Verfahren hat gravierende Nachteile. Bei der Zerkleinerung von Tumoren besteht die Gefahr der Verschleppung von malignen Zellen in die Bauchhöhle. Bei der Entfernung von Zysten kann sich deren Inhalt in die freie Bauchhöhle ergießen, was zu Entzündungen und gegebenenfalls auch zur Verschleppung maligner Zellen führen kann. Im übrigen ist die Zerkleinerung von Gewebe in der Bauchhöhle unter den Bedingungen der minimal invasiven Chirurgie technisch und zeitlich sehr aufwendig. Bei bestimmten Verfahren (bei motorgetriebenen Morcellatoren) besteht eine hohe Verletzungsgefahr für die Organe und Blutgefäße im Bauchraum.

Um diesen Nachteilen zu begegnen, ist eine Vorrichtung bekannt, bei welcher durch die Trokarhülse ein flexibler Sack in die Bauchhöhle eingeführt wird. Der Sack wird vollständig in die Bauchhöhle eingeführt und über Fäden gehalten. Im Innern der Bauchhöhle wird das Gewebe durch die Endöffnung in den Sack verbracht, worauf die Endöffnung mittels durch die Trokarhülse nach außen geführter Fäden zugezogen und verschlossen wird. Der Sack mit dem eingeschlossenen Gewebe kann allerdings bei entsprechendem Durchmesser des Gewebes nicht mehr durch die Trokarhülse herausgezogen werden. Der bestehende Hautschnitt für die Trokarhülse muß daher erweitert werden, um den Sack mit dem Gewebe aus der Bauchhöhle zu entfernen. Die Vorteile der minimal invasiven Operationstechnik gehen dadurch zu einem wesentlichen Teil verloren.

Weiter ist aus der DE-U 92 05 604 eine Vorrichtung der eingangs genannten Gattung bekannt, bei welcher ein Sack mit einer Endöffnung und einer Seitenöffnung verwendet wird. Der Sack wird durch die Trokarhülse soweit in die Bauchhöhle eingeführt, daß sich die Seitenöffnung innerhalb der Bauchhöhle befindet, während die Endöffnung extrakorporal verbleibt. Das zu entfernende Gewebe wird durch die Seitenöffnung in den Sack verbracht, worauf der Sack so weit durch die Trokarhülse herausgezogen wird, daß sich auch die Seitenöffnung extrakorporal befindet. Das Gewebe ist damit innerhalb der Bauchhöhle vollständig von dem Sack umschlossen, so daß bei einer Zerkleinerung des Gewebes in dem Sack keine Gewebeteile oder Flüssigkeit in die freie Bauchhöhle gelangen kann. Zum Zerkleinern des Gewebes müssen dabei geeignete Instrumente durch die Endöffnung des Sackes und die Trokarhülse eingeführt werden.

Der Erfindung liegt die Aufgabe zugrunde, eine Vorrichtung zum Entfernen von Gewebe oder dergleichen aus der Bauchhöhle zur Verfügung zu stellen, welche die Vorteile der minimal invasiven Operationstechnik mit dem sicheren Einschließen des Gewebes in einen Sack vereinigt.

Diese Aufgabe wird erfindungsgemäß gelöst durch eine Vorrichtung mit den Merkmalen des Anspruchs 1.

Vorteilhafte Ausführungen der Erfindung sind in den Unteransprüchen angegeben.

Die Grundidee der Erfindung besteht darin, zum Entfernen des Gewebes einen Sack zu verwenden in dem Mittel zum Zerkleinern des Gewebes vorhanden sind. Der Sack weist außer seiner Endöffnung noch eine Seitenöffnung in dem Wandbereich zwischen der Endöffnung und dem Sackboden auf. Der Sack wird durch die Gewindehülse in die Bauchhöhle eingeführt, wobei er jedoch nicht vollständig in die Bauchhöhle gelangt, sondern seine Endöffnung extrakorporal außerhalb der Gewindehülse bleibt. Durch die Seitenöffnung, die sich bei eingeführtem Sack innerhalb der Bauchhöhle befindet, kann das Gewebe in der Bauchhöhle in den Sack verbracht werden. Anschließend wird der Sack durch die Gewindehülse soweit herausgezogen, bis sich die Seitenöffnung extrakorporal außerhalb der Gewindehülse befindet. Das Gewebe ist damit in einem Aufnahmevolumen am Boden des Sackes vollständig eingeschlossen in der Bauchhöhle, wobei das Sackinnere jetzt nur noch Zugang zum extrakorporalen Raum hat.

Ist der Durchmesser des Gewebes kleiner als der Innendurchmesser der Gewindehülse, so kann der Sack mit dem eingeschlossenen Gewebe durch die Gewindehülse vollständig herausgezogen werden. Weist das in dem Sack eingeschlossene Gewebe jedoch einen größeren Durchmesser auf als der Innendurchmesser der Gewindehülse, so kann das Gewebe in dem Sack zerkleinert werden, da der Innenraum des Sackes über dessen Endöffnung durch die Gewindehülse von außen zugänglich ist. Das das Gewebe aufnehmende Aufnahmevolumen des Sackes, das sich intraabdominal befindet, ist vollständig geschlossen, so daß keine Gefahr besteht, daß bei der Zerkleinerung Gewebsteile, maligne Zellen, Zysteninhalt oder dergleichen in die freie Bauchhöhle gelangen.

Erfindungsgemäß sind in dem Sack Schneidmittel zum Zerteilen von weichen, leicht durchtrennbaren Geweben, angeordnet, die von außen betätigbar sind, um das Gewebe in dem Sack zu durchtrennen. Solche Schneidmittel sind vorzugsweise als Schneid- oder Sägefäden ausgebildet, die das in dem Sack befindliche Gewebe umschlingen und von außen herausgezogen werden können, um das Gewebe zu durchschneiden und zu zerteilen.

Um das Zerkleinern und Zerteilen des Gewebes zu erleichtern, sind im Bereich des Aufnahmevolumens des Sackes in dessen Innerem Fixiermittel vorgesehen, die das Gewebe in dem Sack festhalten, so daß es den Schneidmitteln nicht ausweichen kann. Die Fixiermittel sind vorzugsweise als Fixierstifte im Bereich des Sackbodens ausgebildet, die in das Gewebe eindringen und dieses lagefest halten. Alternativ oder zusätzlich kann zur Fixierung des Gewebes auch die innere Wandoberfläche des Sackes rauh ausgebildet sein. Der Sack kann auch zur Optimierung der Sicherheit doppelwandig, mit zwei übereinander liegenden Wänden ausgebildet sein, welche zur Verstärkung Gewebeeinlagen (dünnes Drahtnetz, Kunststoffnetz, Stoffnetz) enthalten können. Weiterhin können zwischen den doppelten Sackwänden Abstandhalter aus festen, flüssigen oder gasförmigen Stoffen angebracht sein.

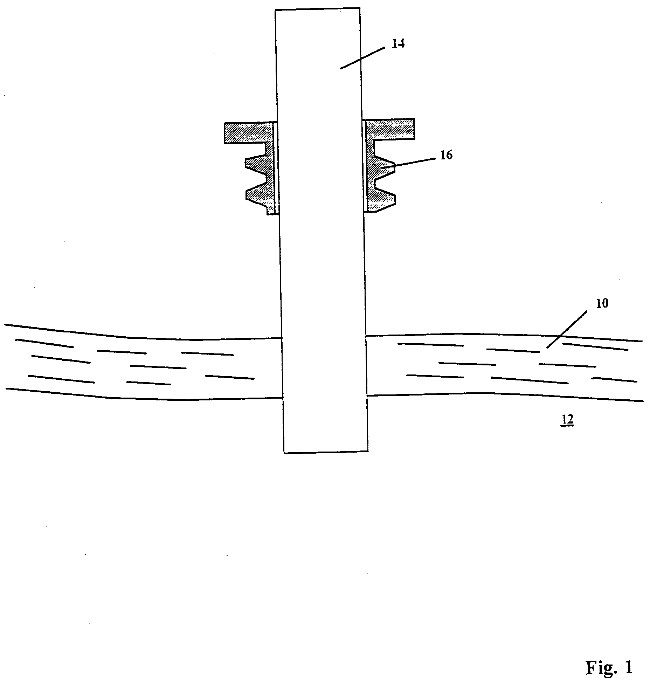

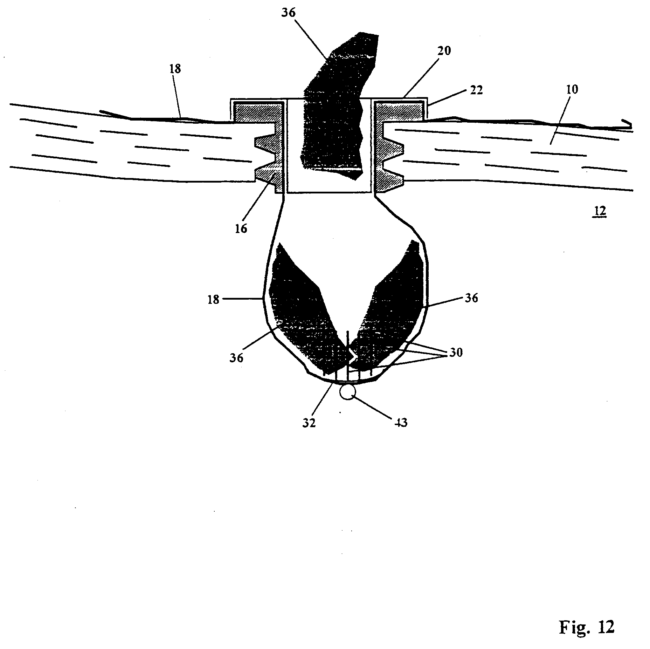

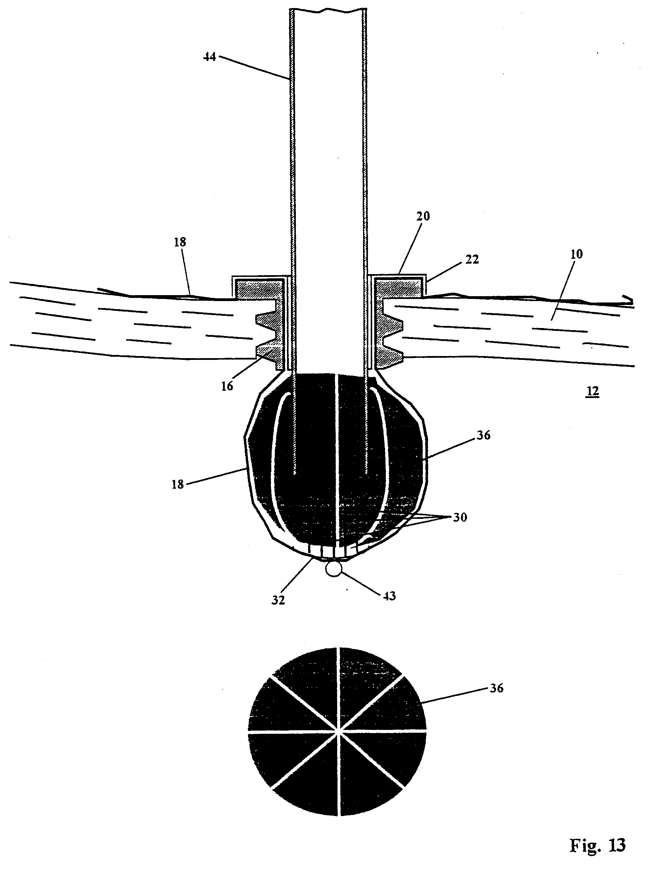

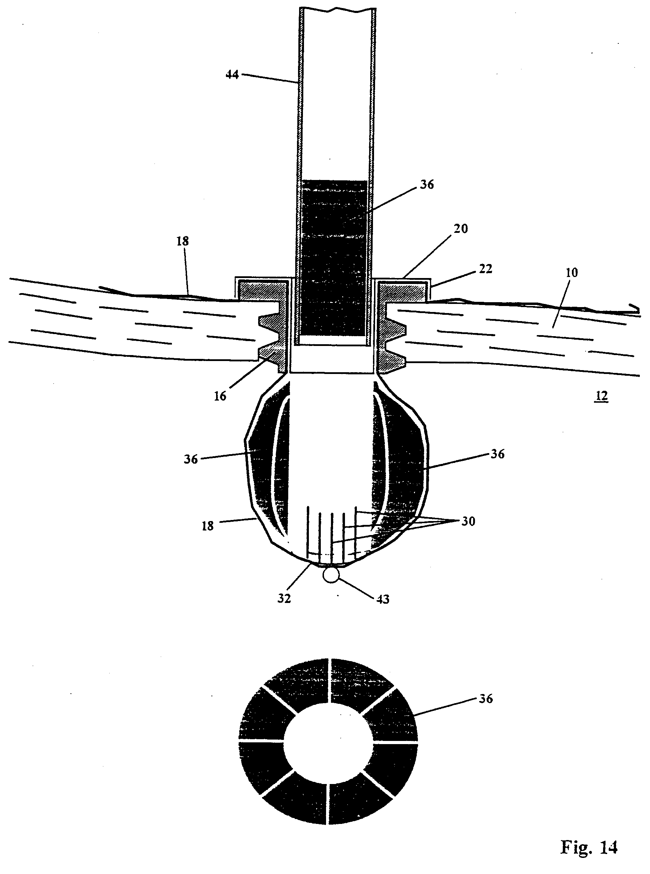

Im folgenden wird die Erfindung anhand eines in der Zeichnung dargestellten Ausführungsbeispieles näher erläutert. Dabei zeigen die Figuren 1 bis 14 die Verwendungsweise der schematisch dargestellten Vorrichtung in aufeinanderfolgenden Schritten.

In Figur 1 ist schematisch die Bauchdecke 10 gezeigt, die die Bauchhöhle 12 verschließt. Die Bauchdecke 10 wird mittels eines Trokars perforiert. Mittels eines Führungsstabes 14, welcher gegen den herkömmlichen Trokar ausgetauscht wird, wird die Bauchdecke 10 offengehalten. Auf dem Führungsstab 14 sitzt eine mit einem Außengewinde versehene Gewindehülse 16.

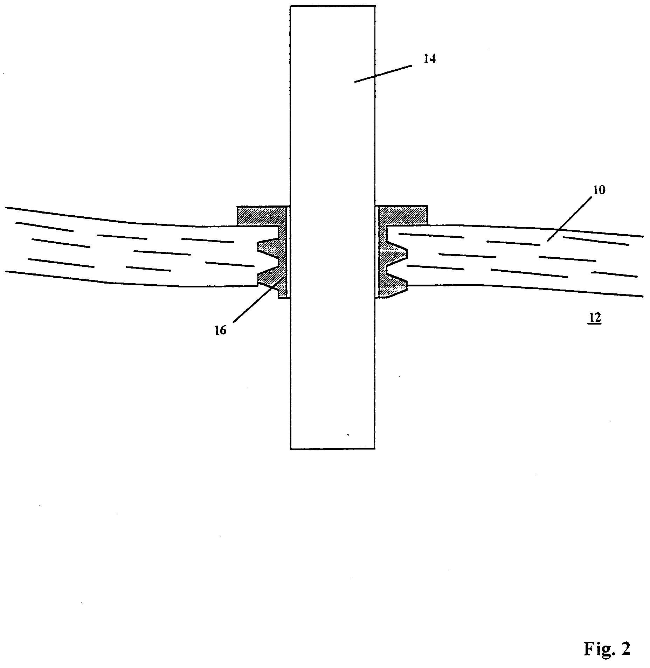

Wie Figur 2 zeigt, wird die Gewindehülse 16 in das Perforationsloch der Bauchdecke 10 eingesetzt und mittels ihres Außengewindes in der Bauchdecke 10 fixiert.

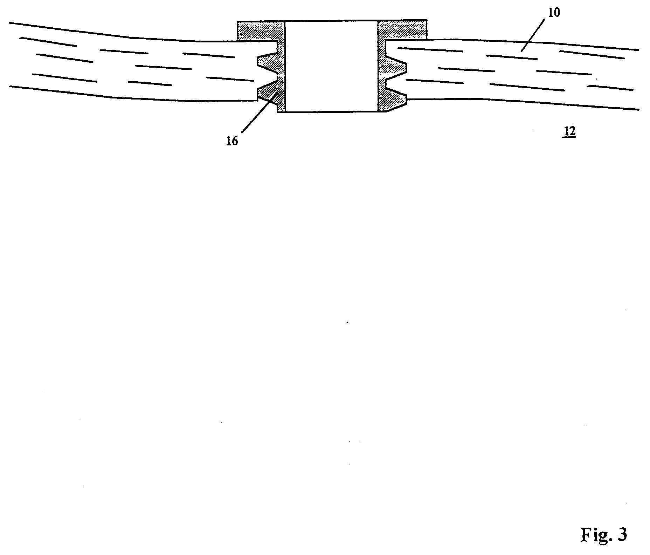

Wie Figur 3 zeigt, wird anschließend der Führungsstab 14 entfernt, so daß die Bauchhöhle 12 über die Gewindehülse 16 zugänglich ist. Gegebenenfalls kann die Gewindehülse 16 in bekannter Weise mit einem Ventil versehen sein.

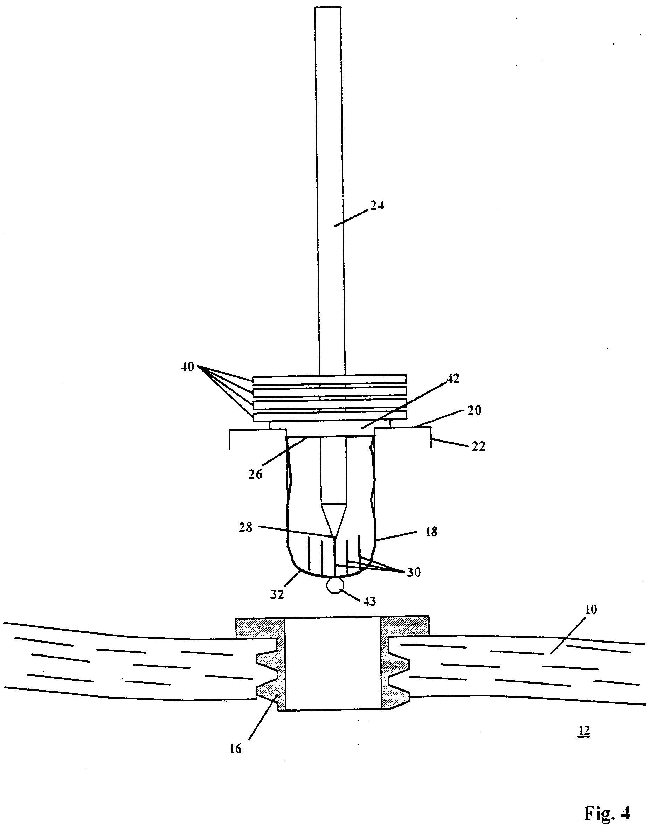

Figur 4 zeigt einen Sack 18 aus einem flexiblen reißfesten und elastischen Kunststoff, der vorzugsweise optisch transparent ausgebildet und zusammengefaltet in einer Einführ- und Sicherungshülse 20 aufgenommen ist. Der Außendurchmesser der Einführ- und Sicherungshülse 20 entspricht dem Innendurchmesser der Gewindehülse 16. Die axiale Länge der Einführ- und Sicherungshülse 20 kann der axialen Länge der Gewindehülse 16 entsprechen. Die Einführ- und Sicherungshülse 20 kann vorzugsweise als einseitig offene Spannhülse ausgeführt sein.

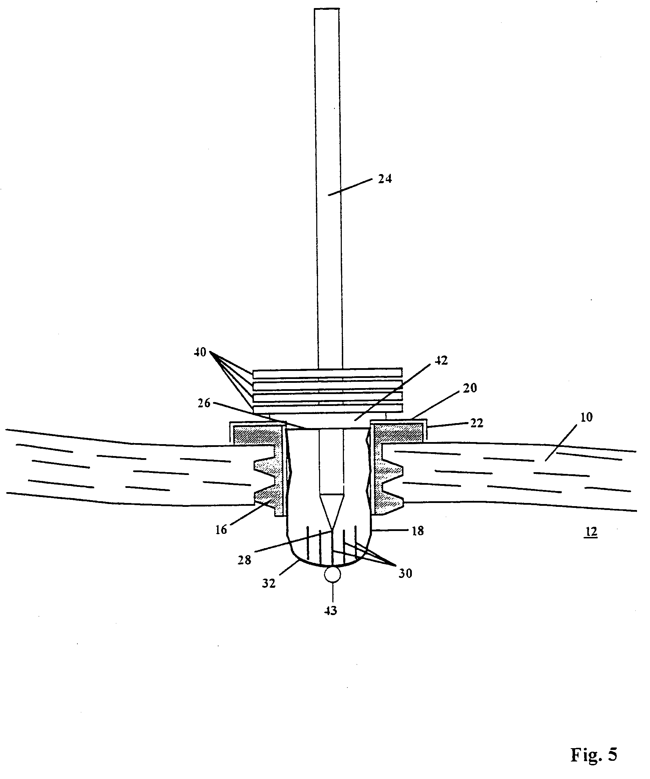

Wie Figur 5 zeigt, wird die Einführ- und Sicherungshülse 20 in die Gewindehülse 16 eingeschoben und stützt sich mit einem endseitigen Flansch 22, welcher gasdicht verschließt, extrakorporal an der Gewindehülse 16 ab. Der Sack 18 ist an seinem extrakorporalen Ende offen, wobei seine Endöffnung 26 an einem Dichtelement 42 der Einführ- und Sicherungshülse 20 lösbar fixiert ist.

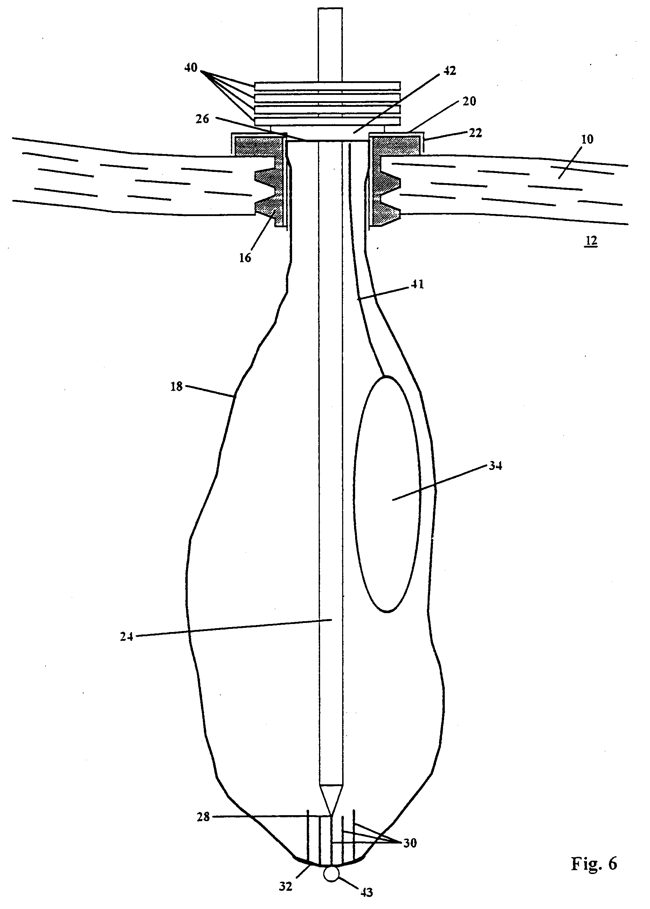

Nach dem Einsetzen der Einführ- und Sicherungshülse 20 in die Gewindehülse 16 wird der Sack 18 in die Bauchhöhle 12 eingeführt, wie Figur 6 zeigt. Hierzu kann ein Führungsstab 24, der durch die Endöffnung 26 in den Sack 18 führt und über eine Sollbruchstelle 28 an einem zentralen Fixierstift 30 des verstärkten Sackbodens 32 befestigt ist, dienen. Durch koaxiales Einschieben des Führungsstabes 24 entfaltet sich der Sack 18 in die Bauchhöhle 12. Alternativ kann der Sack von intraabdominal über eine Manipulationslasche 43 entfaltet werden. Seitlich in der Wandung des Sackes 18 ist eine Seitenöffnung 34 vorgesehen, die sich in der Bauchhöhle befindet, wenn der Sack 18 in die Bauchhöhle 12 eingeführt und entfaltet ist. Ein Spreizring mit Formgedächtnis hält die Seitenöffnung 34 offen, wenn sich der Sack 18 in der Bauchhöhle 12 entfaltet. Eine genaue Plazierung der Seitenöffnung 34 in der Bauchhöhle 12 ist mit dem Führungsstab 24 oder der Manipulationslasche 43 möglich.

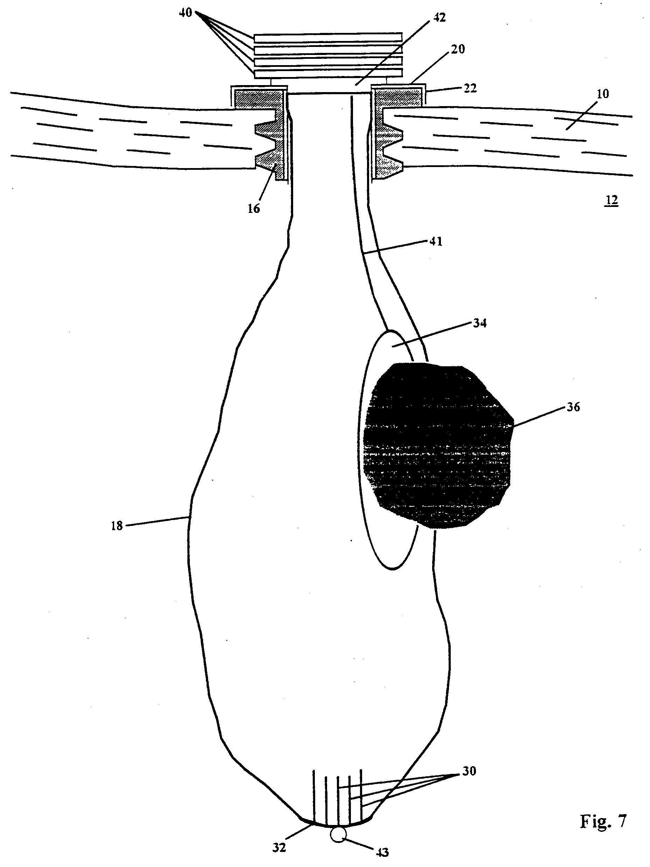

Nach dem Einfügen des Sackes 18 wird der Führungsstab 24 an der Sollbruchstelle 28 abgedreht und aus dem Sack 18 herausgezogen. Wie Figur 7 zeigt, kann nun ein in der Bauchhöhle 12 operativ herausgetrenntes Gewebe 36 durch die Seitenöffnung 34 in den Sack 18 eingebracht werden.

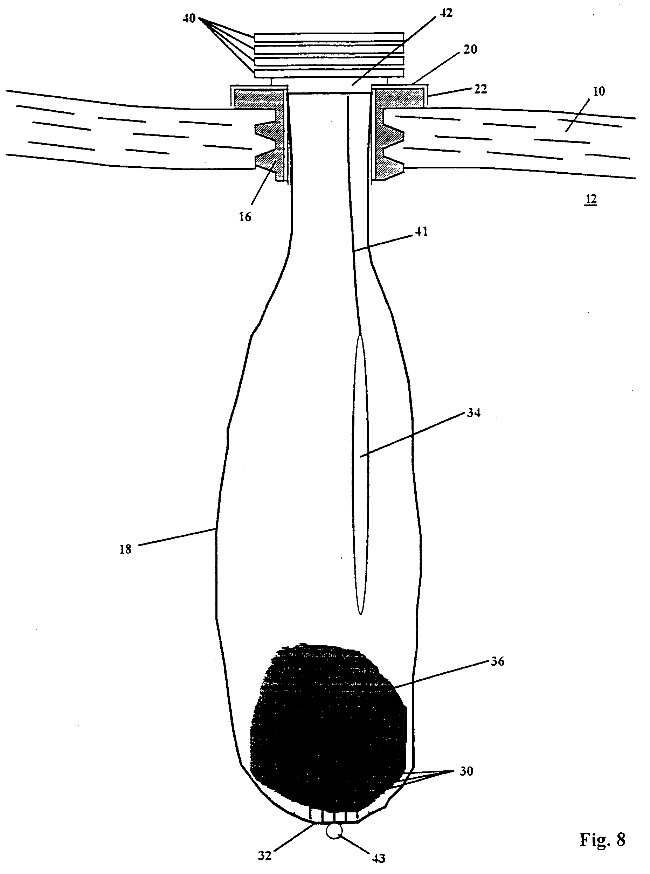

Wie Figur 8 zeigt, gelangt das Gewebe 36 in ein Aufnahmevolumen des Sackes 18, das zwischen dem Sackboden 32 und der Seitenöffnung 34 gebildet ist. Das Gewebe 36 sitzt nun auf Fixierstiften 30 auf, die in den verstärkten Sackboden 32 eingesetzt sind oder an diesem angeformt sind und mit ihren Spitzen in das Innere des Sackes 18 hineinragen. Diese Fixierstifte 30 sind so beschaffen, daß eine Verletzung des Sackes 18 durch diese ausgeschlossen ist.

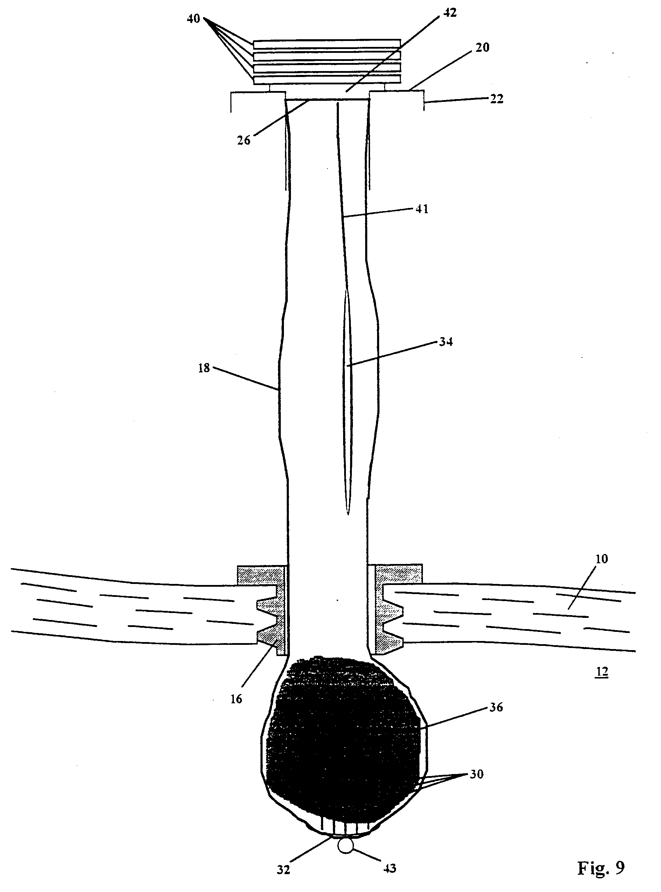

Wie Figur 9 zeigt, werden anschließend die Einführ- und Sicherungshülse 20 und der Sack 18 durch Zug an der Einführ- und Sicherungshülse 20 herausgezogen.

Ist der Durchmesser des Gewebes 36 kleiner als der Innendurchmesser der Gewindehülse 16 so kann der Sack 18 mit dem eingeschlossenen Gewebe 36 vollständig herausgezogen werden, wodurch das Gewebe aus der Bauchhöhle entfernt ist.

Weist das Gewebe 36 jedoch einen größeren Durchmesser auf als der Innendurchmesser der Gewindehülse 16, wie dies in der Zeichnung dargestellt ist, so kann der Sack 18 nur soweit herausgezogen werden, bis das von dem Sack 18 umschlossene Gewebe 36 an dem intraabdominalen Ende der Gewindehülse 16 anliegt, wie dies Figur 9 zeigt. Dadurch wird Gasdichtheit des intraabdominalen Raumes erreicht. Die Seitenöffnung 34 ist in einem solchen Abstand von dem Sackboden 32 entfernt angeordnet, daß die Seitenöffnung 34 bereits vollständig aus der Gewindehülse 16 herausgezogen ist und sich extrakorporal von der Gewindehülse 16 befindet, wenn das eingeschlossene Gewebe 36 an der Gewindehülse 16 zur Anlage kommt. Das Gewebe 36 ist somit durch den Sack 18 vollständig dicht gegen die Bauchhöhle 12 abgeschlossen.

Wenn beim Herausziehen des Sackes 18 das Gewebe 36 an der Gewindehülse 16 zur Anlage kommt, bewirkt die an dem Sack 18 angreifende Zugkraft, daß sich die Fixierstifte 30 des Sackbodens 32 in das Gewebe 36 eindrücken. Das Gewebe 36 wird somit zwischen den Fixierstiften 30 und der Gewindehülse 16 gegen Drehung und seitliche Bewegung fixiert. Zusätzlich kann der Sack 18 durch seine elastische Ausführung das umschlossene Gewebe 36 komprimieren, dieses wird durch den vorherrschenden intraabdominalen Druck (nach CO₂-Insufflation zum Pneumoperitoneum) noch verstärkt.

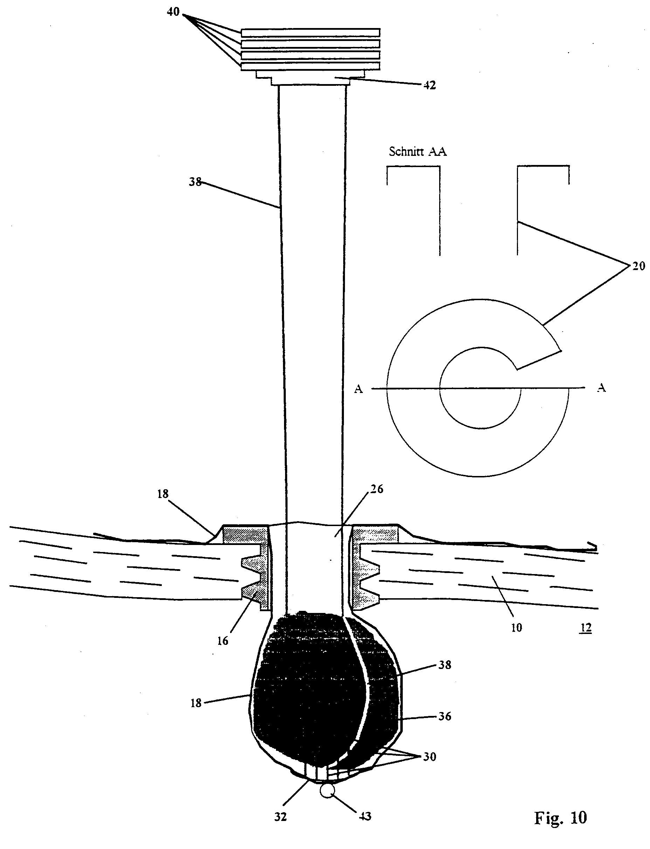

Der Sack 18 kann nun an seinem offenen Ende 26 vom Dichtelement 42 getrennt, entlang von Rißlinien 41 entfaltet und über der Gewindehülse 16 auseinandergebreitet werden, wie dies in Figur 10 gezeigt ist. In dieser Stellung wird der Sack 18 vorzugsweise an der Gewindehülse 16 durch Einschieben der Einführ- und Sicherungshülse 20 (in Gewindehülse 16) fixiert, welche wegen der möglichen Ausführung als einseitig offene Spannhülse den Sack 18 gegen die Innenwand der Gewindehülse 16 drückt und welche an ihrem Flansch 22 einen Klemmechanismus zur Fixation des Sackes 18 hat. Dadurch ist der Sack 18 gegen ein Hineinrutschen in die Bauchhöhle gesichert und das Gewebe 36 wird in der fixierten Stellung gehalten. Die Einführ- und Sicherungshülse 20 kann auch durch zwei Hülsen, eine Einführ- und eine Sicherungshülse, gebildet sein.

Bei leicht schneidbarem, durch die Gewindehülse 16 nicht unmittelbar zurückziehbarem Gewebe 36, z.B. bei Tumoren, Organen oder Organteilen, werden die in den Sack 18 integriert Schneidmittel, wie in dem Ausführungsbeispiel der Zeichnung gezeigt ist, betätigt. Die Schneidmittel sind von außen betätigbar. Eine Zylinderstanze kann nach dem Einsatz der Schneidmittel durch die Einführ- und Sicherungshülse 20 eingeführt werden und zur weiteren Zerkleinerung des Gewebes zum Einsatz kommen.

Diese Schneidmittel bestehen in dem dargestellten Ausführungsbeispiel aus Schneidfäden 38, die in Form einer Schlinge in den Sack 18 eingelegt sind. Die Schlingen der Schneidfäden 38 sind dabei jeweils im wesentlichen in einer axialen Ebene in den Sack 18 eingelegt und an der Innenwand des Sackes 18 durch geeignete Mittel reversibel fixiert. Die Schneidfäden 38 verlaufen am Sackboden 32 zwischen den Fixierstiften 30 und sind auch hier am Sackboden 32 reversibel fixiert. Durch die Anordnung der Schneidfäden 38 zwischen den Fixierstiften 30 ist ein Abrutschen der Schneidfäden 38 vom Gewebe 36 unmöglich und die Schnittrichtung ist definiert. Die Axialebenen der Schlingen der einzelnen Schneidfäden 38 sind dabei gegeneinander im Winkel versetzt. Beispielsweise sind drei jeweils um 120° Grad gegeneinander versetzte Schlingen vorgesehen. Die freien Enden der Schlingen der Schneidfäden 38 sind durch die Endöffnung 26 des Sackes 18 herausgeführt und jeweils an einem Abziehring 40 befestigt. Die Abziehringe 40 der einzelnen Schneidfäden 38 sitzen axial über dem Dichtelement 42 aufeinander. Da sich die Schlingen der Schneidfäden 38 am Sackboden 32 kreuzen, sind die Abziehringe 40 so angeordnet, daß an dem obersten äußersten Abziehring der Schneidfaden 38 befestigt ist, der in dem Kreuzungspunkt der Schneidfäden 38 am Sackboden 32 der oberste ist. Die weiteren Schneidfäden 38 sind mit den Abziehringen 40 entsprechend der Aufeinanderfolge der Schneidfäden 38 am Kreuzungspunkt am Sackboden 32 befestigt.

Durch die Anordnung und Fixierung der Schneidfäden 38 an der Innenwand des Sackes 18 gelangt das Gewebe 36 zwangsläufig in die Schlingen, wenn es durch die Seitenöffnung 34 in den Sack 18 verbracht wird. Die Schlingen der Schneidfäden 38 umschließen das Gewebe 36 jeweils in entsprechend ihrem Winkel gegeneinander versetzten axialen Schneidebenen, wie dies in Figur 10 gezeigt ist.

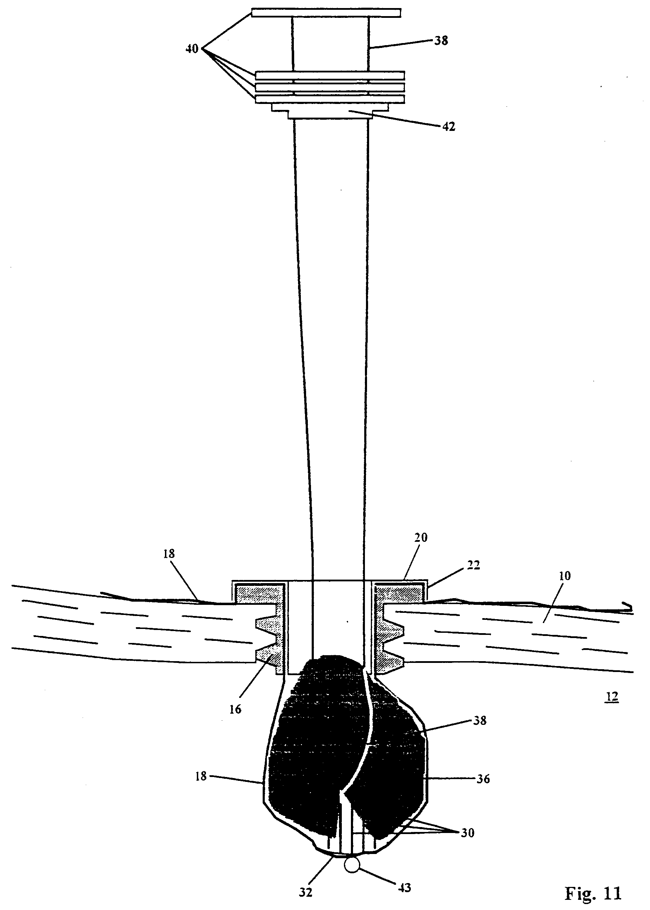

Wie Figur 11 zeigt, wird zunächst der oberste Abziehring 40 abgezogen. Der mit diesem Abziehring 40 verbundene Schneidfaden 38 wird dadurch von seinen Fixierungen an der Wand des Sackes 18 und am Sackboden 32 abgetrennt und schneidend durch das Gewebe 36 gezogen. Da das Gewebe 36 durch die Fixierstifte 30 und die Gewindehülse 16 festgehalten wird, kann das Gewebe 36 der Schneidbewegung des Schneidfadens 38 nicht ausweichen.

Durch aufeinanderfolgendes Abziehen der Schneidfäden 38 mittels der Abziehringe 40 wird das Gewebe 36 in apfelsinenschnitzförmige Teile zerschnitten.

Die Schneidfäden 38 können in vorteilhafter Weise auch eine rauhe, sägeartige Oberfläche zur Optimierung des Schneidvorganges haben.

Nach dem Abziehen sämtlicher Schneidfäden 38 ist das Gewebe 36 in kleine Teile zerteilt, die durch die Gewindehülse 16 bzw. die Einführ- und Sicherungshülse 20 entfernt werden können, wie Figur 12 zeigt. Die Gewebeteile können auch mit der Zylinderstanze 44 ausgestanzt werden (Figur 13). Das ausgestanzte Gewebevolumen (Figur 14) wird durch die umliegenden Gewebeteile aufgefüllt, welche durch die Kompressionswirkung des elastischen Sackes 18 und des Bauchinnenraumes zusammengedrückt werden und nachwandern. Durch mehrmaliges Anwenden der Zylinderstanze 44 können so große Tumore sicher und einfach entfernt werden. Nachdem sämtliche Teile des Gewebes 36 entfernt sind, kann der Sack 18 vollständig herausgezogen werden und die Einführ- und Sicherungshülse 20 und die Gewindehülse 16 werden entfernt.

### Bezugszeichenliste

- 10: Bauchdecke
- 12: Bauchhöhle
- 14: Führungsstab
- 16: Gewindehülse
- 18: Sack
- 20: Einführ- und Sicherungshülse
- 22: Flansch
- 24: Führungsstab
- 26: Endöffnung
- 28: Sollbruchstelle
- 30: Fixierstifte
- 32: Sackboden
- 34: Seitenöffnung
- 36: Gewebe
- 38: Schneidfäden
- 40: Abziehringe
- 41: Rißlinie
- 42: Dichtelement
- 43: Manipulationslasche
- 44: Zylinderstanze

## Patentansprüche

1. Vorrichtung zum Entfernen von Gewebe oder dergleichen aus der Bauchhöhle mit einer in die Bauchdecke (10) einsetzbaren Gewindehülse (16) und mit einem durch die Gewindehülse (16) in die Bauchhöhle (12) einführbaren flexiblen Sack (18), der eine extrakorporal verbleibende Endöffnung (26) und eine Seitenöffnung (34) zum intraabdominalen Einbringen des Gewebes (36) in den Sack (18) aufweist, wobei die Seitenöffnung (34) derart in der Wandung des Sackes (18) zwischen der Endöffnung (26) und dem Sackboden (32) angeordnet ist, daß sich die Seitenöffnung (34) im Inneren dem Bauchhöhle (12) befindet, wenn der Sack (18) bis auf seine extrakorporal von der Gewindehülse (16) verbleibende Endöffnung (26) in die Bauchhöhle (12) eingeführt ist, und daß ein das Gewebe (36) aufnehmendes Aufnahmevolumen des Sackes (18) intraabdominal von der Gewindehülse (16) verbleibt, wenn der Sack (18) soweit herausgezogen ist, daß sich seine Seitenöffnung (34) extrakorporal von der Gewindehülse (16) befindet, wobei zum Zerkleinern des in den Sack (18) eingebrachten Gewebes (36) Schneidfäden (38) oder Sägefäden schlingenförmig in dem Sack (18) angeordnet sind, die zum Zerkleinern des Gewebes (36) durch die Endöffnung (26) des Sackes (18) herausgezogen werden können.

2. Vorrichtung nach Anspruch 1, dadurch gekennzeichnet, daß die durch die Endöffnung (26) des Sackes (18) herausgeführten Enden der Schneidfäden (38) an Abziehringen (40) fixierbar sind.

3. Vorrichtung nach Anspruch 2, dadurch gekennzeichnet, daß mehrere Schlingen von Schneidfäden (38) vorgesehen sind, die mittels axial aneinander angeordneter Abziehringe (40) aufeinanderfolgend abgezogen werden können.

4. Vorrichtung nach einem der vorhergehenden Ansprüche, dadurch gekennzeichnet, daß die Schneidfäden (38) an der Innenwand des Sackes (18) und am Sackboden (32) trennbar festgelegt sind.

5. Vorrichtung nach einem der vorhergehenden Ansprüche, dadurch gekennzeichnet, daß in dem Sack (18) Fixiermittel vorgesehen sind, die das Gewebe (36) in seiner Lage in dem Sack (18) festhalten.

6. Vorrichtung nach Anspruch 5, dadurch gekennzeichnet, daß die Fixiermittel Fixierstifte (30) sind, die am Sackboden (32) angeordnet und nach innen in den Sack (18) gerichtet sind.

7. Vorrichtung nach Anspruch 5, dadurch gekennzeichnet, daß die Fixiermittel durch eine rauhe Wandinnenfläche des Sackes (18) gebildet sind.

8. Vorrichtung nach Anspruch 6, dadurch gekennzeichnet, daß die Schneidfäden (38) zwischen den Fixierstiften (30) hindurchgeführt sind.

9. Vorrichtung nach einem der vorhergehenden Ansprüche, dadurch gekennzeichnet, daß der Sack (18) zusammengefaltet in einer Einführ- und Sicherungshülse (20) aufgenommen ist, die in die Gewindehülse (16) einsetzbar ist, und daß in dem Sack (18) ein trennbar am Sackboden (32) befestigter Führungsstab (24) angeordnet ist, um den Sack (18) aus der Einführ- und Sicherungshülse (20) in die Bauchhöhle (12) einzuführen.

## Claims

1. A device for the removal of tissue or the like from the peritoneal cavity having a threaded bush (16) insertable into the abdominal wall (10) and having a flexible pouch (18) insertable through the threaded bush (16) into the peritoneal cavity (12), which pouch comprises an end opening (26) remaining extracorporeal and a side opening (34) for the intraabdominal introduction of the tissue (36) into the pouch (18), with the side opening (34) being disposed in the wall of the pouch (18) between the end opening (26) and the bottom of the pouch (32) in such a manner that the side opening (34) is located inside the peritoneal cavity (12) when the pouch (18) is introduced into the peritoneal cavity (12) right to its end opening (26), which remains extracorporeally from the threaded bush (16), and in such a manner that a volume of the pouch (18) which contains the tissue (36) remains intraabdominally from the threaded bush (16) when the pouch (18) is withdrawn to the extent that its side opening (34) is situated extracorporeally from the threaded bush (16), to break up the tissue (36) introduced into the pouch (18) cutting threads (38) or saw threads being provided in the form of loops in the pouch (18), which to break up the tissue (36) can be withdrawn through the end opening (26) of the pouch (18).

2. A device according to Claim 1,
**characterised in that** the ends of the cutting threads (38) passed out through the end opening (26) of the pouch (18) can be attached to pull-off rings (40).

3. A device according to Claim 2,
**characterised in that** several loops of cutting threads (38) are provided, which can be removed in succession by means of pull-off rings (40) disposed axially adjacent to one another.

4. A device according to one of the preceding Claims,
**characterised in that** the cutting threads (38) are separably fixed to the inner wall of the pouch (18) and to the bottom of the pouch (32).

5. A device according to one of the preceding Claims,
**characterised in that** provided in the pouch (18) are attachment means which secure the tissue (36) in its position in the pouch (18).

6. A device according to Claim 5,
**characterised in that** the attachment means are attachment pins (30), which are disposed on the bottom of the pouch (32) and are directed inwardly into the pouch (18).

7. A device according to Claim 5,
**characterised in that** the attachment means are formed by a rough inner wall surface of the pouch (18).

8. A device according to Claim 6,
**characterised in that** the cutting threads (38) are guided between the attachment pins (30).

9. A device according to one of the preceding Claims,
**characterised in that** the pouch (18) is contained in the folded state in an introduction and safety sleeve (20), which can be inserted into the threaded bush (16),
**and in that** disposed in the pouch (18) is a pilot rod (24) separably attached to the bottom of the pouch (32) in order to introduce the pouch (18) from the introduction and safety sleeve (20) into the peritoneal cavity (12).

## Revendications

1. Dispositif pour enlever des tissus ou analogues hors de la cavité abdominale, avec une douille filetée (16) pouvant être insérée dans le plafond abdominal (10) et avec un sac flexible (18) pouvant être introduit dans la cavité abdominale (12) en passant à travers la douille filetée (16), sac présentant une ouverture d'extrémité (26) restant en situation extracorporelle et une ouverture latérale (34) destinée à une introduction intra-abdominale des tissus (36) dans le sac (18), l'ouverture latérale (34) étant disposée dans la paroi du sac (18) entre l'ouverture d'extrémité (26) et le fond de sac (32), de manière que l'ouverture latérale (34) se trouve à l'intérieur de la cavité abdominale (12), lorsque le sac (18) a été introduit dans la cavité abdominale (12) jusqu'à son ouverture d'extrémité (26) subsistant en situation extracorporelle vis-à-vis de la douille filetée (16), et en ce qu'un volume réceptacle, recevant les tissus (36), du sac (18) reste en situation intra-abdominale vis-à-vis de la douille filetée (16), lorsque le sac (18) est extrait d'une distance faisant que son ouverture latérale (34) se trouve en situation extracorporelle vis-à-vis de la douille filetée (16), des filaments de découpage (38) ou des filaments de sciage étant disposés en forme de boucles dans le sac (18) en vue de fractionner les tissus (36) introduits dans le sac (18), lesdits filaments pouvant être extraits en passant à travers l'ouverture d'extrémité (26) du sac (18), en vue de fractionner les tissus (36).

2. Dispositif selon la revendication 1,
caractérisé en ce que
les extrémités sorties en passant par l'ouverture d'extrémité (26) du sac (18), des filaments de découpage (38) sont susceptibles être fixées sur des anneaux d'extraction (40).

3. Dispositif selon la revendication 2,
caractérisé en ce que
sont prévues plusieurs boucles de filaments de découpage (38), pouvant être extraites à la suite des unes des autres au moyen d'anneaux d'extraction (40) disposés axialement les uns contre les autres.

4. Dispositif selon l'une des revendications précédentes,
caractérisé en ce que
les filaments de découpage (38) sont fixés, de façon à permettre une séparation, sur la paroi intérieure du sac (18) et sur le fond de sac (32).

5. Dispositif selon l'une des revendications précédentes,
caractérisé en ce que
dans le sac (18) sont prévus des moyens de fixation qui fixent les tissus (36) à leur position dans le sac (18).

6. Dispositif selon la revendication 5,
caractérisé en ce que
les moyens de fixation sont des tiges de fixation (30) disposées sur le fond de sac (32) et tournées vers l'intérieur dans le sac (18).

7. Dispositif selon la revendication 5,
caractérisé en ce que
les moyens de fixation sont constitués par une surface rugueuse intérieure de la paroi du sac (18).

8. Dispositif selon la revendication 6,
caractérisé en ce que
les filaments de découpage (38) sont guidés entre les tiges de fixation (30).

9. Dispositif selon l'une des revendications précédentes,
caractérisé en ce que
le sac (18) est logé à l'état replié dans une douille d'insertion et de sécurité (20) qui peut être insérée dans la douille filetée (16), et en ce que dans le sac (18) est disposée une barre de guidage (24) fixée de façon séparable au fond de sac (32) afin d'insérer dans la cavité abdominale (12) le sac (18) sorti de la douille d'insertion et de sécurité (20).
